# EUROPÄISCHE PATENTSCHRIFT

(11) **EP 2 138 255 B1**
(45) Veröffentlichungstag und Bekanntmachung des Hinweises auf die Patenterteilung: **14.08.2013**
(21) Anmeldenummer: 09007717.3
(22) Anmeldetag: 10.06.2009
(51) Int. Cl.: B23B 51/04, A61B 17/16

(54) **Trepanbohrer**
Trepan
Dispositif de forage à trépan

(30) Priorität: 24.06.2008 DE 102008029920
(43) Veröffentlichungstag der Anmeldung: 30.12.2009
(73) Patentinhaber: Gebr. Brasseler GmbH & Co. KG, D-32657 Lemgo (DE)
(72) Erfinder: Neumeyer, Stefan, Dr., 93458 Eschlkam (DE); Krumsiek, Michael, 32657 Lemgo (DE)
(74) Vertreter: Weber, Joachim

(56) Entgegenhaltungen:
- EP-A1- 1 228 829
- DE-B3-102006 045 508

## Beschreibung

Die Erfindung bezieht sich auf einen Trepanbohrer mit den Merkmalen des Oberbegriffs des Anspruches 1, wie es aus DE 10 2006 045 508 B3 bekannt ist ist.

Im Einzelnen bezieht sich die Erfindung auf einen Trepanbohrer mit einem Schaft, welcher einen Einspannbereich aufweist, sowie einen zylindermantelförmigen, hohlzylindrischen kernbohrerartigen Kopf.

Für die Implantatversorgung bei Kiefern kann es erforderlich sein, Knochenzylinder aus dem Patientenknochen zu gewinnen und diese an geeigneter Stelle zu implantieren. Hierfür werden üblicherweise Trepane eingesetzt. Der Stand der Technik zeigt unterschiedliche Ausgestaltungsformen, welche jeweils einen mit Zähnen versehenen Hohlzylinder oder Kernbohrer umfassen und zusätzlich mit einem Zentrierelement versehen sind. Beispielsweise wird auf die DE 198 01 181 A1 Bezug genommen. Ähnliche Konstruktionen zeigen auch die DE 299 01 804 U1 oder die DE 298 18 798 U1.

Zur Erzeugung der Hohlzylinder durch Trepane oder Trepanbohrer ist es erforderlich, dass diese ein gleichmäßiges und gutes Schnittverhalten aufweisen und ohne thermische Schädigung des Knochens die Entnahme eines exakten Knochenzylinders ermöglichen. Dabei besteht bei den aus dem Stand der Technik bekannten Trepanen die Möglichkeit, dass sich diese durch ungleichmäßige Belastungen seitens des Zahnarztes oder durch unterschiedliche Krafteinleitung durch den zu bearbeitenden Knochen verkanten, verklemmen, in den Knochen fressen oder aus der exakten Schnittführung verlaufen. Was bisher auch immer übersehen wurde ist die Tatsache, dass Verletzungen des Weichgewebes durch diese Trepanbohrer möglich sind. All dies sind unerwünschte Effekte, welche ein qualitatives Bone-Management für die Implantatversorgung negativ beeinflussen.

Die DE 10 2006 045 508 B3 zeigt einen Knochenfräser mit einem Fräserschaft aus einem Metallrohr, an dessen vorderer Kante Zähne ausgebildet sind. Dieser über seine Länge mit einem konstanten Querschnitt versehene Fräser dient dazu, bei mikroinvasiven Operationsmethoden Wirbelbestandteile an seitlichen Fortsätzen eines Wirbelsäulenwirbels abzufräsen. Der zentrische Kanal des Rohres dient dabei zur Entfernung der Knochenspäne und/oder zur Zuführung von Spülflüssigkeit. Ein zylindrischer Knochenzapfen wird hierdurch nicht gewonnen.

Die EP 1 228 829 A1 beschreibt einen Hohlbohrer für Heimwerker, welcher dazu dient, große Löcher, beispielsweise in Wänden oder Holzpaneelen mit geringem Spananfall auszusägen.

Der Erfindung liegt die Aufgabe zugrunde, einen Trepanbohrer der eingangs genannten Art zu schaffen, welcher bei einfachem Aufbau und einfacher, kostengünstiger Herstellbarkeit die Nachteile des Standes der Technik vermeidet und ein gutes Schnittverhalten aufweist und gleichzeitig das Weichgewebe schont.

Erfindungsgemäß wird die Aufgabe durch die Merkmalskombination des Anspruchs 1 gelöst, die Unteransprüche zeigen weitere vorteilhafte Ausgestaltungen der Erfindung.

Erfindungsgemäß ist somit vorgesehen, dass die Verzahnung aus Zähnen aufgebaut ist, welche in Gruppen angeordnet sind. Somit sind in Umfangsrichtung alternierend unterschiedliche Zähne mit unterschiedlicher axialer Zahnhöhe angeordnet. Das bedeutet, dass jeweils in einzelnen Gruppen unterschiedliche Zähne oder unterschiedliche Anordnungen von Zähnen vorgesehen sind. Hierdurch verbessert sich das Schnittverhalten des Trepanbohrers. Es wird insbesondere verhindert, dass dieser unruhig läuft und/oder sich in dem Knochenmaterial verhakt. Durch die erfindungsgemäß vorgesehene Anordnung der einzelnen Zähne in einer Gruppenverzahnung wird ein ruhiges und effizientes Arbeiten ermöglicht.

Der erfindungsgemäße Trepanbohrer ist mit einer Verzahnung gestaltet, durch die beim Einsatz Späne entstehen, die gesammelt werden und anschließend wieder für den Knochenaufbau zum Einsatz kommen können.

Besonders vorteilhaft ist es, wenn erfindungsgemäß benachbart zu einem ersten Zahn, welcher eine größere Zahnhöhe (in axialer Richtung) aufweist, ein weiterer, zweiter Zahn angeordnet ist, welcher eine geringere axiale Zahnhöhe aufweist. Somit folgt auf einen großen Zahn ein kleiner Zahn. Falls sich der "große Zahn" mit der großen Zahnhöhe im Knochenmaterial verhakt oder hängenbleibt, so betrifft dies nicht die gesamte Verzahnung des Trepanbohrers. Vielmehr bewirken die nachfolgenden kleineren Zähne jeweils einen gleichmäßigeren geringeren Schnitt mit geringeren Kräften. Hierdurch ergibt sich ein ruhigerer Lauf des gesamten Trepanbohrers.

Die Schneiden weisen alternierend Höhen-, Tiefen- und Schnittwinkel auf, so dass sich die dadurch erzeugten Späne darin nicht festsetzen, sondern entweder nach innen oder nach außen abgegeben werden.

Der Kopf umfasst eine Gruppenverzahnung, welche einen ersten Zahn sowie zwei weitere Zähne, nämlich einen zweiten Zahn und einen dritten Zahn umfasst. Der erste Zahn ist in Axialrichtung größer ausgebildet, als der zweite und der dritte Zahn. Es ergeben sich somit in Umfangsrichtung mehrere derartige Gruppenverzahnungen.

Besonders günstig ist es, wenn mehrere gleich ausgebildete zweite Zähne benachbart zueinander angeordnet sind. Bei einer derartigen Gruppenverzahnung ist somit ein in Umfangsrichtung und damit in Schnittrichtung führender erster Zahn vorgesehen, dem zumindest zwei kleinere Zähne nachfolgen.

In einer besonders günstigen Ausgestaltung der Erfindung ist vorgesehen, dass die Schneiden der Zähne in unterschiedlicher axialer Anordnung zueinander am distalen Endbereich des Kopfes des Trepanbohrers angeordnet sind. Dies kann bedeuten, dass der größere Zahn axial weiter vorstehend ist, während der zweite bzw. weitere nachfolgende kleinere Zähne axial rückversetzt sind. Selbst bei einem Eindringen des großen Zahnes in das Knochenmaterial erfolgen somit eine Abstützung des Trepanbohrers und eine Fortsetzung des weiteren Zerpanungsvorganges durch die kleineren axial rückversetzten Zähne.

Der Trepanbohrer hat in radialer Richtung gesehen einen relativ breiten Zahnkranz, so dass der Trepanbohrer gleichzeitig auch zur Lagerpräparation bei der chirurgischen Implantatbettbereitung eingesetzt werden kann.

Erfindungsgemäß können die Zähne in unterschiedlicher Weise hinsichtlich ihrer Schneiden und ihrer Schneidgeometrie ausgebildet sein. Besonders günstig ist es, wenn der Spanwinkel in einem Bereich von -20° bis +20° ausgebildet ist.

Auch die Anordnung der Schneiden kann erfindungsgemäß variiert werden. So ist es möglich, die Schneiden aller Zähne oder einiger Zähne der Gruppenverzahnung radial zur Drehachse des Trepanbohrers auszurichten. Es ist jedoch auch möglich, alle Schneiden oder nur einige Schneiden der Zähne der Gruppenverzahnung in einem Winkel zur Radialrichtung anzuordnen. Letzteres kann den Spanfluss und den Abtransport der Knochenspäne verbessern.

Um das Schnittverhalten zu verbessern und um die thermische Belastung des Knochenmaterials durch Reibung an dem zylindrischen Teil des Kopfes zu minimieren, kann es günstig sein, den Zylindermantel des Kernbohrers oder des Kopfes mit Ausnehmungen zu versehen. Diese Ausnehmungen können rund, länglich oder in sonstiger Weise ausgebildet sein. Es können mehrere Reihen von Ausnehmungen in Axialrichtung vorgesehen sein. Günstig kann es sein, wenn zumindest eine Ausnehmung in Umfangsrichtung eine Schneide aufweist, so dass auch der Außenumfang des Kopfes schneidend wirken kann, um ein Verklemmen in dem Knochenmaterial zu vermeiden.

Durch die spezielle Gestaltung der Öffnungen sowie des geschwungenen Übergangsbereiches von Arbeitsteil zum Schaft ist ein weichgewebeschonender Einsatz der Instrumente möglich.

Die Öffnungen im Zylindermantel sind in tangentialer Richtung zur Umdrehungsrichtung beim Einsatz gestaltet, damit sie bei Berührung des Weichgewebes keinen Mitnahmeeffekt zeigen, so dass dadurch eine Reduzierung der Verletzungsgefahr des Weichgewebes entsteht.

In einer besonders günstigen Ausgestaltung der Erfindung ist der Trepanbohrer Teil eines Sets aus zumindest zwei Trepanen. Der oben beschriebene Trepanbohrer dient dabei zur Ausarbeitung eines Knochenzylinders. Ein vor dem Trepanbohrer zu verwendender Zentriertrepan dient zum Zentrieren und zum Erzeugen eines Anschnittbereiches, in welchen dann nachfolgend der eigentliche Trepanbohrer eingesetzt wird.

Innen- und Außendurchmesser der Trepanbohrer der Sets sind so gestaltet, dass sie exakt aufeinander abgestimmt sind und nahtlos ineinander übergehen, so dass der gewonnene Knochenzylinder mit dem eine Nummer größeren Trepanbohrer exakt in die Bohrung hineinpasst, die mit dem um eine Nummer kleineren Trepanbohrer erzeugt wird.

Erfindungsgemäß wurde somit ein Trepanbohrer geschaffen, dessen Verzahnung so gestaltet ist, dass beim Einsatz Späne entstehen, die hinsichtlich ihrer Größe und hinsichtlich der Transportrichtung der Späne so gestaltet und dimensioniert sind, dass sie gut gesammelt und anschließend wieder für den Knochenaufbau zum Einsatz kommen können.

Weiterhin ermöglicht die erfindungsgemäße Verzahnung die Ausbildung eines relativ breiten Zahnkranzes, so dass es möglich ist, den Trepanbohrer in einem System zu verwenden, um eine flächige Vertiefung zu schaffen, die bei der nachfolgenden Versorgung zur Auflage eines Transplantates als Transplantatbett dient.

Der Zentriertrepan umfasst ebenfalls einen an einem Schaft gelagerten Kopf und ist ebenfalls zylindermantelförmig in Form eines Kernbohrers ausgebildet und weist an seinem distalen Randbereich des Hohlzylinders des Kernbohrers Zähne mit Schneiden auf. Zusätzlich ist ein Zentrierelement vorgesehen, welches in bevorzugter Ausgestaltung einstückig mit dem Kopf ausgebildet ist. Die einteilige oder einstückige Ausgestaltung bietet den Vorteil, dass der Zentriertrepan einfacher aufbereitbar ist (leichter gereinigt und desinfiziert werden kann) und dass der Zentriertrepan geringere Rundlauffehler und damit einen gezielten, ruhigeren Lauf aufweist.

Sämtliche Trepane des Sets (Trepanbohrer und Zentriertrepan) sowie gegebenenfalls eine Gingiva-Stanze sind bevorzugterweise aus korrosionsbeständigem Stahl oder aus Titan oder aus Keramik gefertigt.

Der Zentriertrepan umfasst bevorzugterweise ein Zentrierelement, welches in Form eines Spiralbohrers, eines Dreikant-Profils, einer Spitze mit einer Schneide (Eins-Schneider) oder in Form einer Holzbohrerspitze (Stirnbohrer mit Zentrierspitze) ausgebildet ist.

Um den Spanfluss zu verbessern und die Knochenspäne besser abzutransportieren, kann es auch bei dem Zentriertrepan besonders günstig sein, die Schneiden entweder radial oder in einem Winkel zur Radialrichtung anzuordnen.

Im Folgenden wird die Erfindung anhand von Ausführungsbeispielen in Verbindung mit der Zeichnung beschrieben. Dabei zeigt:
- Fig. 1: eine perspektivische Ansicht einer Gingiva-Stanze,
- Fig. 2: eine perspektivische Ansicht eines Zentriertrepans,
- Fig. 3: eine perspektivische eines Trepanbohrers,
- Fig. 4: eine Detailansicht der Verzahnung des Dentalbohrers gemäß Fig. 3,
- Fig. 5: eine Teil-Draufsicht auf die Darstellung der Fig. 4,
- Fig. 6: eine Darstellung, analog Fig. 4, einer abgewandelten Ausgestaltung der Verzahnung,
- Fig. 7-10: unterschiedliche Ausgestaltungen von Ausnehmungen des Kopfes des Trepanbohrers gemäß Fig. 3,
- Fig. 11: eine Ansicht, ähnlich den Fig. 4 und 6, der Verzahnung des Zentriertrepans gemäß Fig. 2,
- Fig. 12: eine stirnseitige Ansicht, analog Fig. 5, der in Fig. 11 gezeigten Verzahnung,
- Fig. 13-16: jeweils Teil-Schnittansichten unterschiedlicher Ausgestaltungsvarianten des Zentriertrepans mit jeweils vergrößerter stirnseitiger Ansicht des Zentrierelements.

In den unterschiedlichen Ausführungsbeispielen sind gleiche Teile mit gleichen Bezugsziffern versehen.

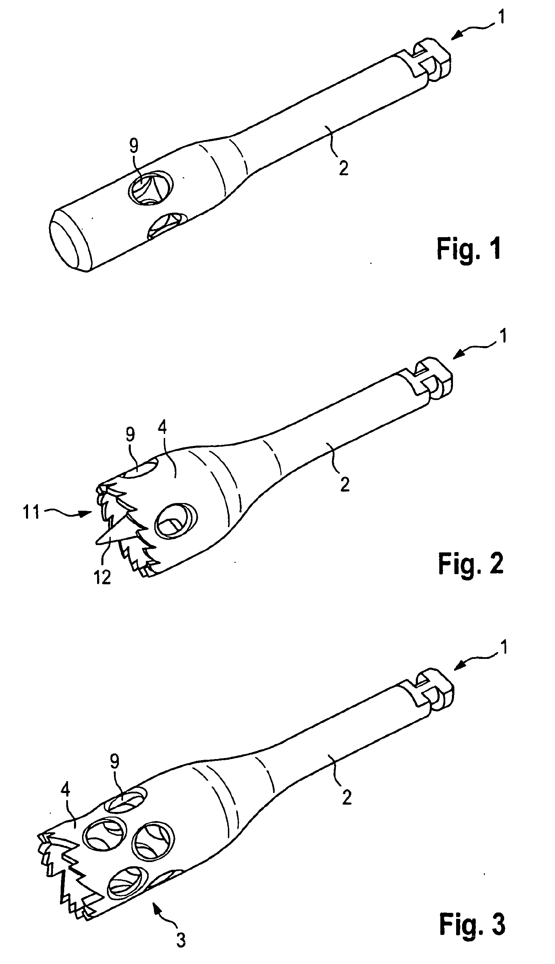

Der in Fig. 3 dargestellte Trepanbohrer umfasst einen Schaft 2 mit einem Einspannbereich 1. Der Einspannbereich 1 ist in üblicher Weise gemäß dem Stand der Technik ausgestaltet und gestattet es, den Trepanbohrer in axial gesicherter Weise zur Drehmomentübertragung in eine Antriebseinheit einzuspannen.

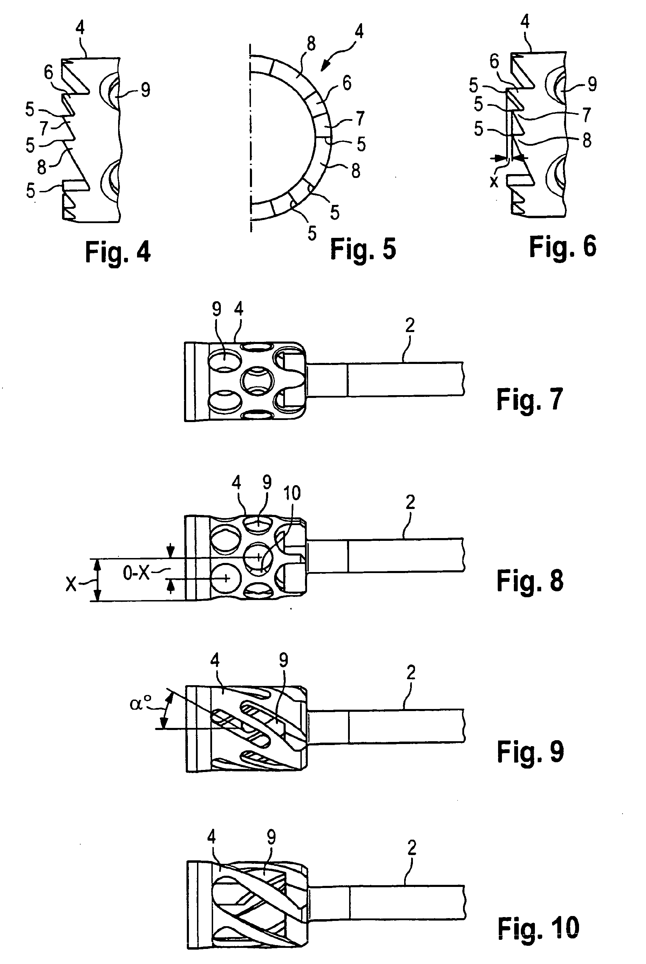

Am anderen Ende des Schaftes 2 ist ein Kopf 3 vorgesehen, welcher einen Hohlzylinder oder Kernbohrer 4 umfasst, der zylindermantelartig ausgebildet ist und an seinem distalen Ende (siehe Fig. 4 und 6) mit Zähnen 6, 7, 8 versehen ist, welche jeweils eine Schneide 5 aufweisen.

Der Kopf 3 des Trepanbohrers ist mit mehreren Ausnehmungen 9 versehen, so wie dies insbesondere in den Fig. 7-10 dargestellt ist. Die Ausnehmungen 9 dienen der besseren Aufbereitung (Reinigung, Sterilisation) sowie der Spanaufnahme und vermindern weiterhin die Reibung am Knochenmaterial, so dass eine Schädigung des Knochenmaterials durch Hitzeentwicklung minimiert werden kann.

In ähnlicher Weise ist der Zentriertrepan 11 gemäß Fig. 2 sowie die Gingiva-Stanze gemäß Fig. 1 mit Ausnehmungen 9 versehen.

Weiterhin erhöhen die Ausnehmungen 9 die Torsionsfestigkeit sowie die Biegefestigkeit und bieten eine bessere Sicht auf das Einsatzumfeld.

Wie die Fig. 7-10 zeigen, können die Ausnehmungen 9 in unterschiedlicher Weise angeordnet und ausgebildet sein, beispielsweise als längliche Schlitze (Fig. 9 und 10) sowie als Reihen von runden Ausnehmungen (Fig. 7 und 8). Wie in Fig. 8 dargestellt, kann die Achse der Ausnehmung 9 auch um einen Betrag zur Radialrichtung seitlich versetzt werden, so dass sich eine Schneide 10 ausbildet, welche einen aggressiven Spanwinkel aufweist. Der seitliche Versatz ist durch den Betrag 0 - X dargestellt, wobei 2*x dem Instrumentendurchmesser entspricht. Im Gegensatz hierzu sind die kreisrunden Löcher der Ausnehmungen 9 gemäß Fig. 7 hinsichtlich ihrer Mittelachse radial zur Drehachse des Trepanbohrers angeordnet.

Wie sich aus den Fig. 4-6 ergibt, umfasst der Kopf 3 eine Gruppenverzahnung, welche einen ersten Zahn 6 sowie zwei weitere Zähne, nämlich einen zweiten Zahn 7 und einen dritten Zahn 8 umfasst. Der erste Zahn 6 ist in Axialrichtung größer ausgebildet, als der zweite und der dritte Zahn 7, 8. Es ergeben sich somit in Umfangsrichtung mehrere derartige Gruppenverzahnungen. In einer abgewandelten Ausgestaltungsform gemäß Fig. 6 sind der zweite Zahn 7 und der dritte Zahn 8 in Axialrichtung zurückversetzt, so dass sie eine um den Betrag x geringere Zahnhöhe aufweisen. Der erste Zahn 6 steht somit um den Betrag x vor. x kann hierbei max. der Zahntiefe des tiefsten Zahnes entsprechen.

Es versteht sich, dass erfindungsgemäß auch andere Kombinationen derartiger Gruppenverzahnungen ausgebildet sein können.

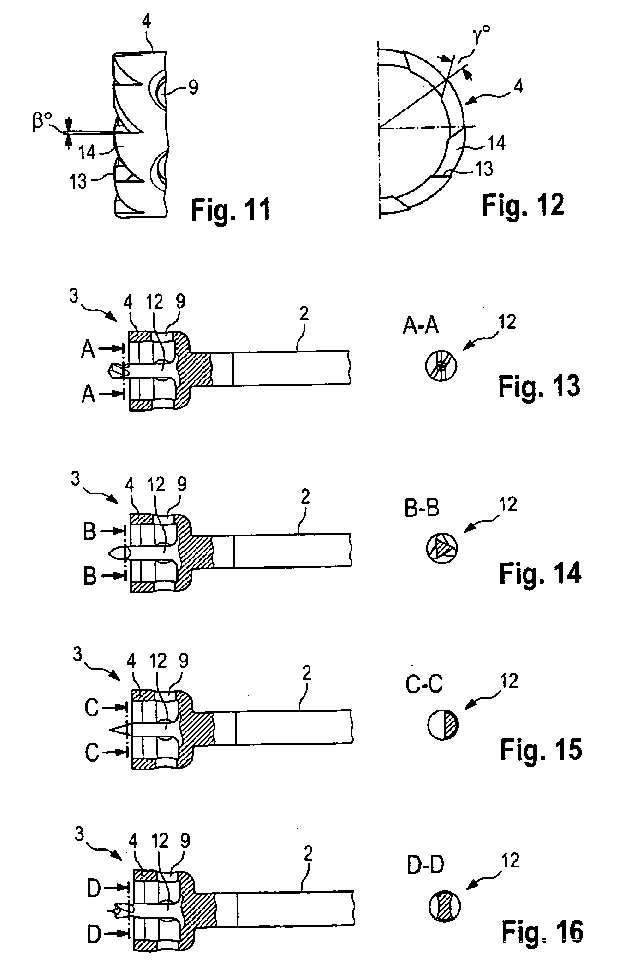

Insgesamt ergibt sich durch die Ausgestaltung der Verzahnung ein gut kontrollierbares, ruhiges und effizientes Arbeiten des Trepanbohrers. Auch bei dem Kopf 3 des Trepanbohrers ist es möglich, die Schneiden 5 entweder radial anzuordnen oder um einen Winkel y zur Radialrichtung zu neigen, so wie dies schematisch in Fig. 12 dargestellt ist. Der Winkel y beträgt zwischen -30° und 30°.

Der Spanwinkel ist bevorzugterweise in einem Bereich von -20° bis +20° ausgebildet.

Die Fig. 11-16 zeigen einen als Zentriertrepan ausgestalteten Trepanbohrer. Dessen Kopf 3 weist ebenfalls einen hohlzylindrischen Kernbohrer 4 auf, welcher an seiner distalen Stirnseite mit Zähnen 14 versehen ist, welche Schneiden 13 umfassen. Wie in Fig. 12 gezeigt, können die Schneiden 13 auch um einen Winkel y von -30° bis 30° zur Radialrichtung geneigt angeordnet sein.

Der Zentriertrepan 11 weist ein einstückig oder einteilig ausgebildetes Zentrierelement 12 auf, welches, wie in den Fig. 13-16 dargestellt, das distale Ende des Kernbohrers 4 überragt und als Spiralbohrer (Fig. 13), als Dreikantprofil (Fig. 14), als Einschneider (Fig. 15) oder als Holzbohrerspitze (Fig. 16) ausgebildet sein kann.

Auch bei dem Zentriertrepan führen die Ausnehmungen 9 zu einer geringeren seitlichen Reibung bei optimiertem Sichtfeld. Durch den Einsatz des Zentriertrepans kann der Entnahmeort des Knochenzylinders genau definiert werden, es wird eine ringkreisförmige Nut erzeugt, in welche der Trepanbohrer (Fig. 3) anschließend eingesetzt und zum weiteren Bohren geführt werden kann.

Die in Fig. 1 gezeigte Gingiva-Stanze oder Schleimhautstanze ist gemäß dem Stand der Technik ausgebildet, die Ausnehmungen 9 dienen ebenfalls der besseren Aufbereitung.

Durch die erfindungsgemäße Ausgestaltung des Trepanbohrers und die Möglichkeit, seinen Innen- und Außendurchmesser, bedingt durch die Form der Verzahnung, entsprechend zu wählen und exakt aufeinander abzustimmen und nahtlos ineinander überzugehen, ergibt sich somit die bereits erwähnte Möglichkeit, einen Knochenzylinder passend zu erzeugen und einzusetzen.

Durch die alternierende Ausgestaltung der Schneiden wird erfindungsgemäß sichergestellt, dass sich die erzeugten Späne nicht in der Verzahnung festsetzen, sondern nach innen oder nach außen abgegeben werden.

Die Öffnungen im Zylindermantel des chirurgischen Trepanbohrers sind in tangentialer Richtung zur Umdrehungsrichtung gestaltet, damit sich bei Berührung des Weichgewebes kein Mitnahmeeffekt ergibt. Hierdurch wird die Verletzungsgefahr des Weichgewebes reduziert.

Die Trepane des Sets sind somit hinsichtlich der Kombination von Schnittbreite und Formkongruenz aufeinander abgestimmt und optimiert. Hierdurch ist es möglich, ein Transplantatbett zu schaffen. Mittels eines ersten Trepanbohrers wird dabei eine kreisringförmige Ausfräsung in dem Knochenmaterial erzeugt. Mittels eines zweiten Trepanbohrers, welcher exakt durch die Zentrierspitze (Zentrierelement 12) geführt wird, ist es möglich, eine angrenzende kreisringförmige Nut zu erzeugen. Somit kann durch Verwendung mehrerer, aufeinander abgestufter Trepanbohrer eine ebene Transplantatbett-Fläche geschaffen werden. Ein restlich zentrisch noch verbleibender geringfügiger zylindrischer Stumpf kann mittels eines Löffels entfernt werden. Somit ergibt sich ein ebenes, ringförmiges Transplantatbett, in welches passgenau ein Knochenzylinder, welcher durch einen Trepanbohrer des Sets an anderer Stelle entnommen wurde, eingesetzt werden kann. Somit ist es nicht erforderlich, zusätzliche Planfräser oder ähnliches zu verwenden.

### Bezugszeichenliste

- 1: Einspannbereich
- 2: Schaft
- 3: Kopf
- 4: Hohlzylinder/Kernbohrer/Zylindermantel
- 5: Schneide
- 6: Erster Zahn
- 7: Zweiter Zahn
- 8: Dritter Zahn
- 9: Ausnehmung
- 10: Schneide der Ausnehmung
- 11: Zentriertrepan
- 12: Zentrierelement
- 13: Schneide des Zentriertrepans
- 14: Zahn des Zentriertrepans

## Patentansprüche

1. Chirurgischer Trepanbohrer mit einem mit einem Einspannbereich (1) versehenen Schaft (2) sowie mit einem Kopf (3), welcher einen im Wesentlichen zylindermantelförmigen Kernbohrer (4) umfasst, an dessen distalem Endbereich jeweils mit einer Schneide (5) versehene Zähne (6, 7, 8) ausgebildet sind, wobei in Umfangsrichtung alternierend Zähne (6, 7, 8) mit unterschiedlicher axialer Zahnhöhe angeordnet sind, **dadurch gekennzeichnet, dass** die Zähne eine Gruppenverzahnung von jeweils drei Zähnen (6, 7, 8) bilden, wobei ein erster, in Schnittrichtung vorne liegender Zahn (6) in Axialrichtung größer ausgebildet ist und wobei zwei in Schnittrichtung nachfolgende Zähne (7, 8) gleich ausgebildet sind.

2. Chirurgischer Trepanbohrer nach Anspruch 1, **dadurch gekennzeichnet, dass** die Schneiden des zweiten (7) und des dritten (8) Zahns in Axialrichtung gleich angeordnet sind.

3. Chirurgischer Trepanbohrer nach Anspruch 1 oder 2, **dadurch gekennzeichnet, dass** die Schneide (5) des ersten Zahns (6) distal weiter vorstehend als die Schneide (5) des zweiten und des dritten Zahns (7, 8) angeordnet ist.

4. Chirurgischer Trepanbohrer nach einem der Ansprüche 1 bis 3, **dadurch gekennzeichnet, dass** der Zylindermantel (4) mit fensterartigen Ausnehmungen (9) versehen ist, wobei die Kanten der Ausnehmungen im Außenbereich nichtschneidend ausgeführt sind.

5. Chirurgischer Trepanbohrer nach einem der Ansprüche 1 bis 4, **dadurch gekennzeichnet, dass** der Übergangsbereich vom Kopf (3) zum Schaft (2) zur Weichgewebeschonung konvex-konkav verlaufend und/oder nicht-scharfkantig ausgeführt ist.

6. Chirurgischer Trepanbohrer nach einem der Ansprüche 1 bis 5, **dadurch gekennzeichnet, dass** zumindest ein Zahn (6, 7, 8) mit einem Spanwinkel in einem Bereich von -20° bis +20° versehen ist.

## Claims

1. A surgical trepan drill comprising a shaft (2) provided with a clamping portion (1), and a head (3) that comprises a substantially cylinder barrel shaped core bit (4) the distal end portion of which has formed thereon teeth (6, 7, 8), each provided with a cutting edge (5), circumferentially alternating teeth (6, 7, 8) being arranged at a different axial tooth height, **characterized in that** the teeth form a group toothing of three respective teeth (6, 7, 8), wherein a first tooth (6) positioned at the front in cutting direction is made larger in axial direction and wherein two teeth (7, 8) following in cutting direction are formed identically.

2. The surgical trepan drill of claim 1, **characterized in that** the cutting edges of the second (7) and the third (8) tooth are arranged identically in axial direction.

3. The surgical trepan drill of claim 1 or 2, **characterized in that** the cutting edge (5) of the first tooth (6) is distally protruding further than the cutting edge (5) of the second and the third tooth (7, 8).

4. The surgical trepan drill of any of claims 1 to 3, **characterized in that** the cylinder barrel (4) is provided with window-type recesses (9), wherein the edges of the recesses are configured to be non-cutting in an external region.

5. The surgical trepan drill of any of claims 1 to 4, **characterized in that** a transitional area from the head (3) to the shaft (2) extends convex-concave and/or is made without a sharp edge to preserve the soft tissue.

6. The surgical trepan drill of any of claims 1 to 5, **characterized in that** at least one tooth (6, 7, 8) is provided with a cutting angle in a range of -20° to +20°.

## Revendications

1. Dispositif de forage à trépan chirurgical avec un arbre (2) pourvu d'une zone de serrage (1) ainsi qu'avec une tête (3) qui comporte un dispositif de forage à carotte (4) sensiblement en forme d'enveloppe cylindrique, sur la zone d'extrémité distale duquel sont réalisées des dents (6, 7, 8) respectivement pourvues d'une lame (5), des dents (6, 7, 8) avec une hauteur axiale différente étant disposées en alternance dans le sens périphérique, **caractérisé en ce que** les dents forment une denture groupée de respectivement trois dents (6, 7, 8), une première dent (6) se trouvant devant dans le sens de coupe étant réalisée plus grande dans le sens axial et deux dents (7, 8) consécutives dans le sens de coupe étant réalisées de manière identique.

2. Dispositif de forage à trépan chirurgical selon la revendication 1, **caractérisé en ce que** les lames de la deuxième (7) et la troisième (8) dents sont disposées de manière identique dans le sens axial.

3. Dispositif de forage à trépan chirurgical selon la revendication 1 ou 2, **caractérisé en ce que** la lame (5) de la première dent (6) est disposée plus en avant dans le sens distal que la lame (5) de la deuxième et troisième dents (7, 8).

4. Dispositif de forage à trépan chirurgical selon l'une quelconque des revendications 1 à 3, **caractérisé en ce que** l'enveloppe cylindrique (4) est pourvue d'évidements (9) de type fenêtre, les arêtes des évidements dans la zone extérieure étant réalisées de manière non tranchante.

5. Dispositif de forage à trépan chirurgical selon l'une quelconque des revendications 1 à 4, **caractérisé en ce que** la zone de transition de la tête (3) à l'arbre (2) est réalisée de manière convexe ou concave et/ou de manière non tranchante pour épargner le tissu mou.

6. Dispositif de forage à trépan chirurgical selon l'une quelconque des revendications 1 à 5, **caractérisé en ce qu'**au moins une dent (6, 7, 8) est pourvue d'un angle de coupe compris dans une plage de -20° à +20°.
